# EUROPEAN PATENT SPECIFICATION

(11) **EP 2 008 622 B1**
(45) Date of publication and mention of the grant of the patent: **05.09.2012**
(21) Application number: 08158866.7
(22) Date of filing: 24.06.2008
(51) Int. Cl.: A61F 2/46, A61L 27/38

(54) **Kit with Osteogenic Prosthesis**
System mit osteogener Prothese
Kit avec prothèse ostéogène

(30) Priority: 27.06.2007 US 823407
(43) Date of publication of application: 31.12.2008
(73) Proprietor: DePuy Products, Inc., Warsaw, IN 46581 (US)
(72) Inventor: Gundlapalli, Ramarao V, Leesburg, IN 46538 (US); Cassell, Dennis R Jr, Fort Wayne, IN 46804 (US); Schnieders, Barry A, Plymouth, IN 46563 (US); Edgar, Derek R, Fort Wayne, IN 46814 (US); Patil, Karuna, Chicago, IL 60605 (US)
(74) Representative: Belcher, Simon James

(56) References cited:
- EP-A- 1 396 241
- WO-A-2006/071758
- US-A1- 2006 153 001
- US-B2- 6 723 131

## Description

The present invention relates generally to the field of medical device implants, and more particularly, to an implant used with a biological factor.

Patients who suffer from the pain and immobility caused by osteoarthritis and rheumatoid arthritis have an option of joint replacement surgery. Joint replacement surgery is quite common and enables many individuals to function properly when it would not be otherwise possible to do so. Artificial joints are usually comprised of metal, ceramic and/or plastic components that are fixed to existing bone.

Such joint replacement surgery is otherwise known as joint arthroplasty. Joint arthroplasty is a well-known surgical procedure by which a diseased and/or damaged joint is replaced with a prosthetic joint. In a typical total joint arthroplasty, the ends or distal and or superior portions of the bones adjacent to the joint are resected or a portion of the distal and/or superior part of the bone is removed and the artificial joint is secured thereto.

There are known to exist many designs and methods for manufacturing implantable articles, such as bone prostheses. Such bone prostheses include components of artificial joints such as elbows, hips, knees and shoulders.

Bone grafting is widely used to treat fractures, non-unions and to induce arthrodeses. Autogenous cancellous bone, which is taken from one site in the patient and implanted in another site in the patient, is currently the most effective bone graft. Autogenous cancellous bone provides the scaffolding to support the distribution of the bone healing response, and progenitor cells which form new cartilage or bone.

Accordingly, alternatives to autografts have been developed. Several purified or synthetic materials, including ceramics, biopolymers, processed allograft bone and collagen-based matrices have been investigated or developed to serve as substitutes for autografts. The FDA has approved a porous coral-derived synthetic hydroxyapatite ceramic for use in contained bone defects. A purified collagen/ceramic composite material is also approved for use in acute long bone fractures. The synthetic graft materials have also been used as carriers for progenitor cells. When the above composite materials are implanted into skeletal defects, progenitor cells differentiate into skeletal tissue.

In some instances, composite implants are made by combining a synthetic graft material in a cell suspension with a similar or lesser volume obtained from a bone marrow aspirate. The numbers of progenitor cells present in 1 ml of bone marrow varies widely between patients from about 100 cells to 20,000 cells. This represents a mean of about one in 20,000 to one in 40,000 of the nucleated cells in a bone marrow aspirate. For example, iliac crest bone marrow tends to have concentrations of progenitor cells which are higher than those found in long bone marrow.

Mesenchymal stem cells or human bone marrow stromal stem cells are defined as pluripotent progenitor cells with the ability to generate cartilage, bone, muscle, tendon, ligament and fat. These primitive progenitors exist postnatally and exhibit stem cell characteristics, namely low incidence and extensive renewal potential. These properties in combination with their developmental plasticity has generated interest in the potential use of mesenchymal stem cells to heal or repair damaged tissues. In essence mesenchymal stem cells could be cultured to expand their numbers then transplanted to the injured site or after seeding in/on shaped biomimetic scaffold to generate cellular rich tissue constructs.

Thus, an alternative approach for skeletal repair is the selection, expansion and modulation of osteoprogenitor cells in combination with a conductive or inductive scaffolds to support and guide regeneration together with judicious selection of osteotropic growth factors.

Human bone marrow osteoprogenitors can be isolated and enriched using selective markers, such as STRO-1, from a CD34+ fraction and these cells can be readily expanded, indicating their potential for marrow repopulation. Current work is centred on the isolation and expansion of mesenchymal stem cells and the development of protocols for their use in a variety of tissue engineering applications.

While the iliac crest is the optimum source of bone graft, other sites for harvesting bone graft are also possible. For example the large long bones in the skeleton, for example the femur and the tibia may be used as a source for bone graft.

The effectiveness of any biologic bone graft depends on its inherent characteristics and cellular constituents, particularly the subset of stem cells that are capable of growing new bone. It has long been recognized that bone marrow has inherent osteogenic (bone growth) potential due to the presence of osteoprogenitor cells (a type of cell that helps make new bone). If delivered in an appropriate matrix, these cells have been shown to contribute substantially to bone formation at the grafted or implanted site.

The use of autogenous cancellous bone has long been an established means for treating bone fractures in defects. As an alternative, synthetic options have been developed. Synthetic options include several purified natural collagen derivatives, ceramics, and polymer materials. The more successful of these materials can attribute their success to chemistry and structure, in that they all closely resemble some fundamentals aspects of the native bone composition and architecture. Collagen derived materials moulded into scaffolds provide structural support and an abundance of the principal protein compound of bone. Calcium phosphate, hydroxyapatite, and tricalcium-phosphate based bone grafts provide a biomemetic matrix and interconnected porosity conducive to osteoprogenitor interactions, proliferation and vascular flow. While structurally and chemically these synthetic materials have proven a successful alternative to autograft.

Accordingly, several techniques were developed that provide for the increased osteoconductivity of bone graft materials. All these processes involve, in some form, mixing iliac crest bone marrow harvest with the bone graft material, given that bone marrow contains both bioactive factors and osteoprogenitor cells for bone development. This technique involves plating a suspension of bone marrow cells onto tissue culture dishes, culturing the cells in a select medium for one or more days to achieve an enhanced population of progenitor cells, and then detaching the cells from the tissue culture dishes to provide a cell suspension containing an increased population of progenitor cells. These methods have been successful in particular because osteoprogenitor cells selectively adhere to the calcium phosphate and hydroxapatite matrix of bone grafts. Examples of such techniques are disclosed in US-6723131, US-6049026, US-5824084 and US-A-2004/0191897.

Composite implants can be made by soaking synthetic ceramic carriers in this progenitor cell enriched suspension. Unfortunately, this method of preparing composite implants is very time consuming. Moreover, if the original progenitor culture cells are derived from bone marrow aspirates obtained from the patient, the patient must undergo multiple invasive procedures; one procedure to remove his or her bone marrow, and another procedure at that time or on a later date to implant the composite graft. Consequently, the patient may be exposed to anaesthesia more than once.

Another technique has also been developed to produce a composite bone graft matrix having the benefits of the culture method, but is not so time consuming and does not require multiple invasive procedures. In this technique, a composite matrix having an enriched population of progenitor cells is produced by contacting a particular volume of matrix material with an excess volume of bone marrow aspirate (see US-6723131, US-6049026, US-5824084 and US-A-2004/0191897). In that technique, bone marrow aspirate containing progenitor cells is passed through a porous matrix material having a surface which selectively bonds progenitor cells, thus retaining the progenitor cells within the matrix and allowing excesses of other cells (such as blood cells and other nucleated marrow-derived cells) to pass through. The now progenitor cell-enriched graft matrix is implanted in a patient.

In parallel to bone graft development there has been significant research and validation for use of porous metal metallic structures for superior orthopaedic implant fixation, in-growth and subsequent long term success. It is now widely agreed that the high coefficient of friction characteristic of surface metallic porous coatings improves initial implant fixation and provides an optimal pore size for bony in-growth. It is also known that many metallic structures exist throughout which there is a gradient of porosity, increased porosity toward the bone implant interface which upon implant optimizes the volume and density of bone ingrowth into the structure. However, metallic structures are inherently limited to be biocompatible while bone graft materials can achieve osteoconductivity.

A useful porous coating can be provided by a plurality of sintered beads which are fastened to the surface of a substrate, for example. Products having a particular porous coating are available from DePuy Orthopaedics Inc under the trade mark Porocoat. Details of such a coating are disclosed in US-3855638.

Accordingly, a preferred porous coating consists of a plurality of small discrete particles of metallic material bonded together at their points of contact with each other to define a plurality of connected interstitial pores in the coating. A preferred porous coating is a composite of small discrete particles of metallic material bonded together resulting in a porous structure which still has significant mechanical stability. The particles are of the same metallic material as the metallic material from which the substrate is formed. It is essential that this be the case otherwise corrosion at the substrate-coating interface may occur due to a cell action with body fluids.

The metallic material from which the substrate and coating are formed is one which is not corroded or otherwise degraded by the body fluids of the patient. Examples of suitable materials include austenitic stainless steel, titanium, titanium alloys and cobalt alloys. The cobalt alloy sold under the trade mark VITALLIUM has been found to be especially useful.

The depth of the porous coating on the surface of the substrate and the ratio of depth of coating to depth of substrate may vary over a wide range between essential limits. The lower limit of thickness is about 100 µm, which is the thickness of surface coating required to sustain bone tissue ingrowth with good mechanical interlocking in the pores, generally equivalent to about 2 to 3 monolayers of particles, while the upper limit of thickness is about 1,000 µm which is dictated by the strength considerations discussed above. Typically, a depth of about 500 µm is used on about a 6.4 mm (0.25 inch) round substrate, using from +325 to -100 mesh particle coatings.

While the materials may be a solid metal with a porous coating as described above, the materials may alternatively be fully porous materials. Such fully porous materials may include that sold under the trade mark TRABECULAR METAL by Zimmer Technology Inc of 345 East Main Street, Warsaw, Indiana 46580 USA, and other titanium structures. Other fully porous materials include isostatically moulded foam metal or similar biocompatible, metallic load-bearing implant components.

Metals are not osteoconductive, but surface modifications, such as by adding hydroxyapatite [HA] coatings and calcium phosphate (Ca₂PO₄) coatings have attempted to increase biocompatibility and bone graft for enhanced fixation.

A known method for preparing a composite bone graft is disclosed in EP-A-1 396 241 and in US-5824084 and is described below with reference to FIG. 1. The apparatus, shown generally as 10A, comprises a porous, biocompatible, implantable substrate 12A, a container 14A, for holding substrate 12A, a reservoir 16A for holding the bone marrow aspirate suspension, a first fluid flow regulator 18A, a second fluid flow regulator 20A, and an effluent collector 22A. Prior to preparation of the composite bone graft, all of the components of the apparatus are sterilized. Following removal of top 23A, the bone marrow aspirate suspension is introduced into reservoir 16A. Then fluid flow regulator 18A is opened to allow the bone marrow aspirate suspension to flow out of reservoir 16A and into opening 30A in removable top 24A of container 14A and onto substrate 12A.

As the suspension enters substrate 12A, fluid flow regulator 20A which is attached to tip 34A of container 14A is opened to permit the effluent of the bone marrow aspirate suspension to flow through porous member 32A, through opening 36A of container 14 and into effluent collector 22A.

Reservoir 16A and removable top 24A are then detached from container 14 and the improved composite bone marrow graft is then removed from container 14A.

Another known method for preparing a composite bone graft is disclosed in US-6723131 is described below with reference to FIG 2. A composite biocompatible implantable matrix 10B is placed into a matrix container, which container is most preferably a column 12B. (Matrix 10B may be packed tightly or loosely, depending on the material and its structure) Column 12B can be provided having a multitude of interior volumes suitable to accommodate the necessary volume of matrix for a particular graft. As used herein, a volume of matrix (or matrix volume) refers to the excluded volume of a nonporous solid having external dimensions identical to those of the particular matrix. For example, a column having an internal volume of 5, 10, 15, 20, 25, or 30 cm³ or some other internal volume, can be provided to accommodate various matrix volumes. Preferably, column 12B has an interior diameter of 0.5 cm, more preferably at least 1.0 cm. Preferably the interior diameter is not more than 3.0 cm, more preferably not more than 2.0 cm, more preferably not more than 1.5 cm. Preferably, column 12B has a length at least 1.5, more preferably at least 2, most preferably at least 3, times greater than its interior diameter. End caps 14B are removably attached to column 12B via threaded connections, snap connections, or any other known connecting means.

Optionally, end caps 14B can be provided with a screen or membrane effective to allow aspirate 20B to pass there through, while retaining particles of matrix 10B. Preferably, such a membrane has openings of at least 20, preferably at least 30, preferably at least 40 µm in diameter.

A bone marrow aspirate 20B (preferably containing an anticoagulant) is obtained via known means, preferably from the patient. Aspirate 20B is then loaded into a first loading syringe 28B. Initially, aspirate 20B contains progenitor cells and other nucleated cells in a ratio between 1:20,000 and 1:40,000.

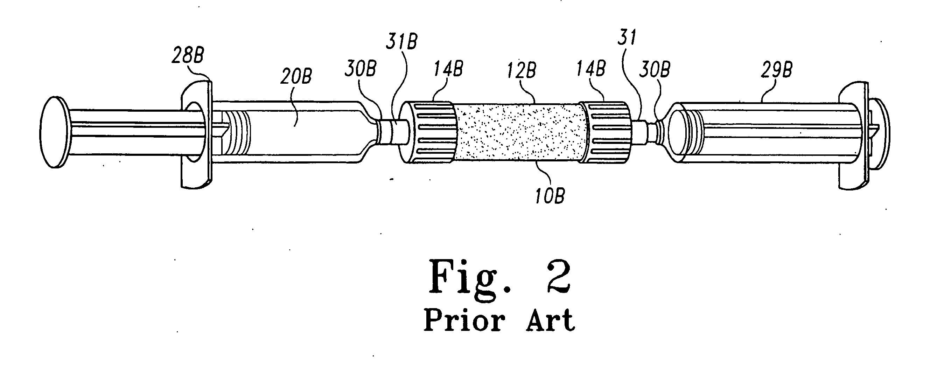

The aspirate also contains platelets, red blood cells and serum (including molecules which are soluble or suspended in serum). Loading syringe 28B is provided with a syringe connector 30B adapted to mate with end cap connector 31B, to provide fluid communication between the respective interior volumes of loading syringe 28B and column 12B. A second loading syringe 29B is similarly provided with a syringe connector 30B adapted to mate with end cap connector 31B. As seen in FIG. 2 first and second loading syringes 28B and 29B are then attached at opposite ends of column 12B to end caps 14B via the above-described connectors, thus providing fluid communication between the interior volumes of first loading syringe 28B, column 12B, and second loading syringe 29B.

First loading syringe 28B is then plunged, delivering aspirate 20B into column 12B where aspirate 20B flows through or contacts matrix 10B prior to being collected at the opposite end of column 12B in second loading syringe 29B. Contacting the bone marrow aspirate and the matrix to provide an enriched matrix can be done by flowing the aspirate through the matrix, incubating the matrix in the aspirate, or by other means known in the art. Alternatively, aspirate 20B is contacted with matrix 10B by any known means to provide an enriched matrix. Progenitor cells advantageously and selectively adhere to the surface of matrix 10B, and hence are retained within the matrix while excesses of other cells, such as blood cells and other marrow-derived nucleated cells, flow relatively freely through the matrix and are collected in second loading syringe 29B.

Accordingly, it is prudent to integrate successful bone graft materials with porous orthopaedic devices where long term success of the implant is directly dependant on the strength of the bone-implant interface. It is desirable to have an in-growth supplement intended for use with a porous metallic scaffold which can either be part of the bulk structure or a surface coating on an orthopaedic device that is adjacent to the bone interface. It is important also to have the tools and the methodology for application of this ingrowth supplement to the porous structure. A method that encompasses preparation of an ingrowth supplement with competitive retention and selectivity for osteoprogenitor cells and, subsequent application to a porous metallic scaffold without loss of this biological efficacy is specifically sought.

The present invention can be used in a new methodology for preparing novel composite orthopaedic structures. This novel structure is composed of a metallic porous structure and is seeded with an ingrowth supplement. The metallic porous structure may be a solid metal material coated with a porous coating such as provided by a plurality of bonded particles (especially by sintering, for example as in implant components which are sold under the trade mark Porocoat) or HA (hydroxyapatite). The term in-growth supplement refers any material that may serve to promote the growth of bone. In-growth supplements may include both organic and inorganic compounds. For example, an in-growth supplement may be in the form of bone marrow aspirate. Alternatively, the in-growth supplement may be in the form of a combination of bone marrow aspirate and granulated calcium phosphate, tri-calcium phosphate or collagen -derived bone graft, with optional inclusion of a known anti-infective or anti-microbial or growth factor agent. The composite orthopaedic structure will be prepared by passing bone marrow aspirate through the porous bone graft material to create a viscous bioactive composite that has an enriched supply of osteoprogenitor cells into the open porous structure of the metal.

This procedure can be performed interoperatively, in order to reduce time, stress, pain, discomfort, and cost to the patient or patient. Interoperative procedures also support an increased population of viable cells within the bone marrow aspirate, as it reduces time between aspiration from patient, preparation of the novel composite material, and implant into the physiological environment. The improved composite orthopaedic implant prepared by these methodologies has a greater number of osteoprogenitor cells, biological stimulants, osteoconductive matrix, while maintaining mechanical properties conferred by an implant's bulk metallic composition necessary for bone repair and replacement.

Features of this invention include the application of an ingrowth supplement to a porous metallic scaffold. This process significantly contributes to the capacity of porous metallic structures to form a healthy and long term, mechanically sound interface with bone at the implant site. This presentation of invention includes tools and implants that are specifically designed to apply these methods to orthopaedic implants, such as those for knees, hips, shoulders, ankles and other joints, including for example, acetabular cups, of a partially or fully porous nature. The implants may be in the form of a total joint replacement or partial joint replacement and may, for example, apply to wedges and augments that are used in revision surgeries. However, it should be noted, that this technology is not limited to any particular orthopaedic device. One process and apparatus that may be used with the present invention is disclosed in US-6723131.

The present invention can be used in a method for preparing porous metallic structures intended for implantation into, in place of, or adjacent to bone. The methodology includes preparation of an ingrowth supplement for seeding into porous structures. The invention also relates to the process of applying the ingrowth supplement to a three dimensional metallic scaffold for a novel composite orthopaedic implant providing superior fixation resulting in long term success. The term in-growth supplement refers any material that may serve to promote the growth of bone. In-growth supplements may include both organic and inorganic compounds. For example, an in-growth supplement may be in the form of bone marrow aspirate. Alternatively, the in-growth supplement may be in the form of a combination of bone marrow aspirate and granulated calcium phosphate, tri-calcium phosphate or collagen derived bone graft. The invention also relates to the tools necessary for seeding this ingrowth supplement into a porous metallic scaffold of an orthopaedic implant. The invention is well suited to all implants and in particular those implants where fixation may be an issue. Such implants include those placed in patients with weak bone structure and those known as revision implants that are placed in patients to replace a surgically removed implant. Such implants include partially or completely porous acetabular cups for total arthroplasty, extensive pelvic and hip trauma, and for revision cases.

The invention provides a kit for performing joint arthroplasty, as defined in claim 1.

Apparatus provided by the invention can be used in a method for providing joint arthroplasty comprising:
providing a prosthetic component having a porous surface;
providing a fixture for surrounding the component, the fixture including a surface for closely conforming to the portion of the surface of the first portion of the component having the porous surface, the fixture having an inlet and an opposed outlet
providing a biological compound including stem cells;
injecting at least a portion of the biological compound into the inlet of the fixture;
advancing the biological compound through the porous surface of the component toward the outlet of the fixture;
expelling at least a portion of the biological compound out the outlet of the fixture;
resecting a portion of a bone; and
implanting the prosthetic component onto the bone with a at least a portion of the porous surface in contact with the bone.

According to the present invention, there is also provided a prosthetic implant for securing to bone to form an articulating joint. The implant is prepared by a process which includes the steps of preparing a porous surface on a component to form a prosthetic implant, placing the prosthetic implant including the porous surface in a fixture closely conforming to the porous surface of the implant and having an inlet and an opposed outlet, and directing a biological compound including stem cells into the inlet, through the porous surface of the implant and out of the outlet to form a prosthetic implant having a porous surface coated with a portion of the biological compound.

According to another aspect of the present invention there is provided a kit for performing joint arthroplasty. The kit includes a component having a first portion with a surface adapted to be fixedly secured to bone. At least a portion of the surface of the first portion has a porous structure. The kit also includes a biological compound including stem cells adapted to be at least partially dispersed in the porous structure of the surface of said first portion. The kit also includes a vessel for containing the biological compound and a fixture for surrounding the component. The fixture includes a surface for closely conforming to the portion of the surface of the first portion of said component having a porous structure. The fixture has an inlet and an opposed outlet. The kit also includes a device for advancing the biological compound from the vessel, into the inlet of the fixture, and out of the outlet of the fixture.

According to the present invention there is also provided an instrument for use in applying a biological compound to the porous surface of a prosthetic implant to be implanted onto a surface of a bone. The instrument includes a vessel for containing the biological compound and a fixture for surrounding the component. The fixture includes a surface for closely conforming to the portion of the surface of the first portion of the component having the porous structure. The fixture has an inlet and an opposed outlet. The instrument also includes a device for advancing the biological compound from the vessel, into the inlet of the fixture, and out of the outlet of the fixture.

Apparatus provided by the invention can be used in a method for providing joint arthroplasty. The method includes the steps of providing a prosthetic component having a porous surface and providing a fixture for surrounding the component. The fixture includes a surface for closely conforming to the portion of the surface of the first portion of the component having the porous surface. The fixture has an inlet and an opposed outlet. The method also includes the steps of providing a biological compound including stem cells and injecting at least a portion of the biological compound into the inlet of the fixture. The method also includes the steps of advancing the biological compound through the porous surface of the component toward the outlet of the fixture and expelling at least a portion of the biological compound out the outlet of the fixture. The method also includes the steps of resecting a portion of a bone and implanting the prosthetic component onto the bone with at least a portion of the porous surface in contact with the bone.

According to the present invention, there is also provided a prosthetic implant for securing to bone to form an articulating joint. The implant is prepared by a process which includes the steps of preparing a component with a porous surface to form a prosthetic implant and placing the prosthetic implant including the porous surface in a cavity of a container. The process also include the steps of directing a biological compound including stem cells into the cavity of the container and submersing the porous surface of the implant with biological compound. The process also include the steps of sealing the container with the implant submersed in the biological compound and submitting the cavity of the container to a pressure greater than ambient pressure to urge the biological compound into the porous surface of the prosthetic implant.

For example, a method for performing joint arthroplasty using the invention can include the steps of providing a prosthetic component having a porous surface. Injecting a needle into the iliac crest of a patent to remove a biological compound including stem cells. The method further includes the step of injecting at least a portion of the biological compound into the porous surface of the prosthetic component. The method further includes the step of resecting a portion of a bone and implanting a prosthetic component into the bone of the patient with at least a portion of the porous surface in contact with the bone.

The technical advantages of the present invention further include the ability to reduce infection during an orthopaedic surgery. For example, a prosthetic implant for securing to bone to form an articulating joint can include a first component having a first portion including a surface with a porous structure. The implant further includes a biological component including an anti-infective and or an anti-microbial agent in the biological compound. The biological compound is positioned in the porous structure and the implant is implanted with the porous structure positioned against bone.

The technical advantages of the present invention further include the ability to improve the fixation of an implant to the bone of a patient after arthroplasty. For example, a prosthetic implant for securing to bone to form an articulating joint can include a first component having a first portion including a surface with a porous structure. The implant further includes a biological component including a growth factor in the biological compound. The growth factors may include pyrophosphate, statins, proton-pump inhibitors, parathyroid hormones and vitamin K. The biological compound is positioned in the porous structure and the implant is implanted with the porous structure positioned against bone.

The prosthetic implant further includes a biological compound including stem cells at least partially dispersed into porous structure of the surface of the surface of the first portion of said first component. Thus the present invention provides for improved bone fixation by providing a biological compound to improve the bone fixation of the porous structure to bone.

The technical advantages of the present invention further include the ability to use standard porous implants. For example, a prosthetic implant for securing to bone to form an articulating joint can be in for example the form of a standard hip or knee or other joint component. At least one of the components includes a portion that has a porous structure. A biological component including stem cells is at least partially dispersed in the porous structure of the surface of the implant with the porous structure positioned against bone.

The technical advantages of the present invention further include the ability to use fully porous implants. For example, a fully porous prosthetic implant for securing to bone to form a scaffold for use in arthroplasty can for example be a fully microporous titanium, for example of the kind marketed under the trade mark TRABECULAR METAL by Zimmer Technology Inc. The prosthetic implant may be in for example the form of a scaffold for a hip or knee or other joint arthroplasty. The implants may be in the form of a total joint replacement or partial joint replacement and may, for example, apply to wedges and augments that are used in revision surgeries. At least one of the components includes a portion that has a porous structure. A biological component including stem cells is at least partially dispersed in the porous structure of the surface of the implant with the porous structure positioned against bone.

The technical advantages of the present invention further include the ability to improve the adherence of biological compounds to porous implants with chemical treatment. For example, a chemical treatment can be added to a porous prosthetic implant for securing to bone for use in arthroplasty. The treatment may for example be adding silicone to the oxide layer of the implant. This process is known as silanisation. The prosthetic implant may be in for example for a hip or knee or other joint arthroplasty. At least one of the components includes a portion that has a porous structure. A biological component including stem cells is at least partially dispersed in the porous structure of the surface of the implant with the porous structure positioned against bone. Thus the present invention provides the ability to improve the adherence of biological compounds to porous implants.

The technical advantages of the present invention further include the ability to improve the adherence of biological compounds to porous implants with biological compounds. For example, a coating can be added to a porous prosthetic implant for securing to bone for use in arthroplasty. The coating serves to improve the adherence of biological compounds to porous implants. The prosthetic implant may be in for example for a hip or knee or other joint arthroplasty. At least one of the components includes a portion that has a porous structure. A biological component including stem cells is at least partially dispersed in the porous structure of the surface of the implant with the porous structure positioned against bone.

The technical advantages of the present invention further include the ability to apply a biological component to an orthopaedic implant surface. For example, a fixture can be provided which includes a portion for receiving the prosthetic implant. The fixture can further include a portion for receiving the biological compound. The fixture may include an arm or level which when energized advances the biological component across the porous structure to apply the biological compound to the implant. Thus, the present invention provides for a biological component that is quick to apply to the porous structure of an orthopaedic implant.

The technical advantages of the present invention further include the ability to provide for enhanced concentration of stem cells in a biological compound. For example, a method for providing joint arthroplasty can include a step of providing an orthopaedic component having a porous structure and providing a biological component including stem cells. The biological compound is processed to enhance the concentration of stem cells using technology such as that provided in US-6723131, US-6049026, US-5824084 and US-A-2004/0191897, in which bone marrow aspirate containing progenitor cells is passed through a porous matrix material having a surface which selectively bonds to progenitor cells, thus retaining the progenitor cells within the matrix and allowing excesses of other cells (such as blood cells and other nucleated marrow-derived cells) to pass through. Thus the present invention provides for enhanced concentration of stem cells in the biological compound.

Embodiments of the invention are described below by way of example with reference to the accompanying drawings, in which:

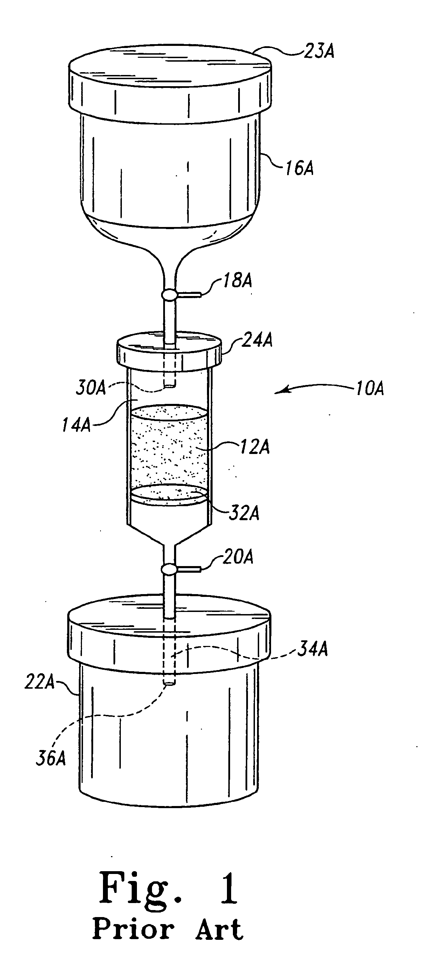

FIG.1 is a plan view of a prior art system of applying a solution to a substrate;
FIG 2 is a plan view of a prior art system of applying a solution to a substrate;
FIG. 3 is a perspective view of a prosthetic implant according to an embodiment of the present invention;
FIG. 4 is a plan view of the prosthetic implant of FIG. 5 in position in the acetabulum;
FIG. 5 is a plan view of a hip prostheses assembly for use in the kit of the present invention;
FIG. 6 is a plan view of a hip stem prostheses for use in the kit of the present invention;
FIG. 7 is a perspective view of another hip cup prostheses for use in the kit of the present invention;
FIG. 8 is a plan view of a knee prostheses for use in the kit of the present invention;
FIG. 9 is a perspective view of a kit including a mixing and delivery system and the prosthesis of FIG. 3 according to another embodiment of the present invention;
FIG. 10 is a medial/lateral view of a patient with an aspiration needle show in position on the iliac crest;
FIG. 11 is a perspective view of a needle for collecting bone marrow aspirate for use in the implant of FIG. 3;
FIG. 12 is a perspective view of a device for processing the bone marrow aspirate of FIG. 11 according to the present invention;
FIG. 13 is a cross sectional view of the device of FIG. 12;
FIG. 14 is a perspective view of the a fixture for holding the prosthetic implant of FIG. 3 for applying the biological agent to the implant using the device of FIG. 12 according to another embodiment of the present invention;
FIG. 15 is a cross-sectional view of FIG. 14 along the line 15-15 in the direction of the arrows;
FIG. 16 is a plan view of the a device for processing the bone marrow aspirate of FIG. 9 according to another embodiment of the present invention;
FIG. 17 is a perspective view of one of the syringes of the device of FIG. 16;
FIG. 18 is a perspective view of the device of FIG. 16;
FIG. 19 is a plan view of a fixture for holding the acetabular component of FIG. 3 for use in the mixing and delivery system shown in FIG. 11 with the fixture in a open position;
FIG. 20 is a plan view of the fixture of FIG. 14 with the fixture in a closed position;
FIG. 21 is a flow chart of a surgical procedure according to another embodiment of the present invention;
FIG. 22 is a flow chart of another surgical procedure according to another embodiment of the present invention; and
FIG. 23 is a schematic diagram of another device for applying a biological coating to a component to form an implant according to another embodiment of the present invention

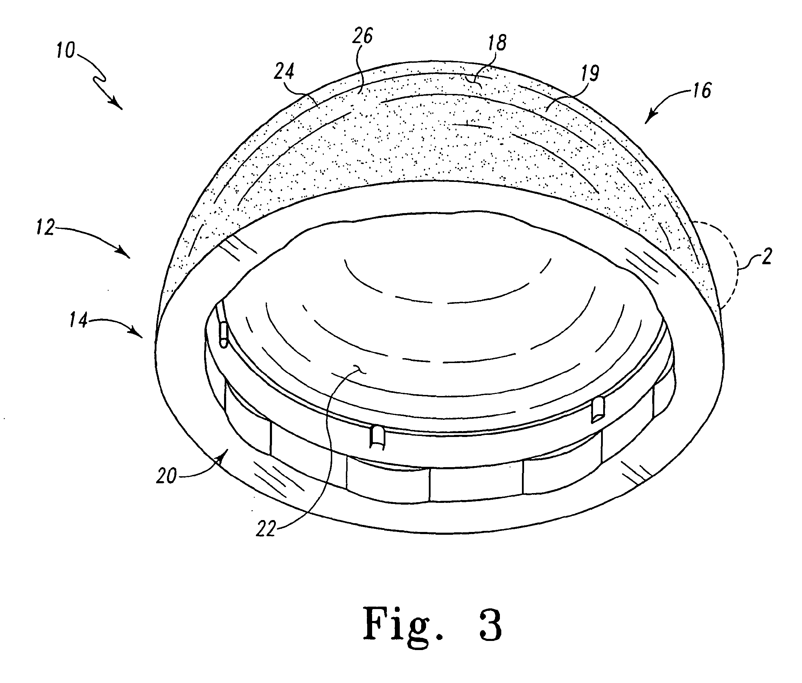

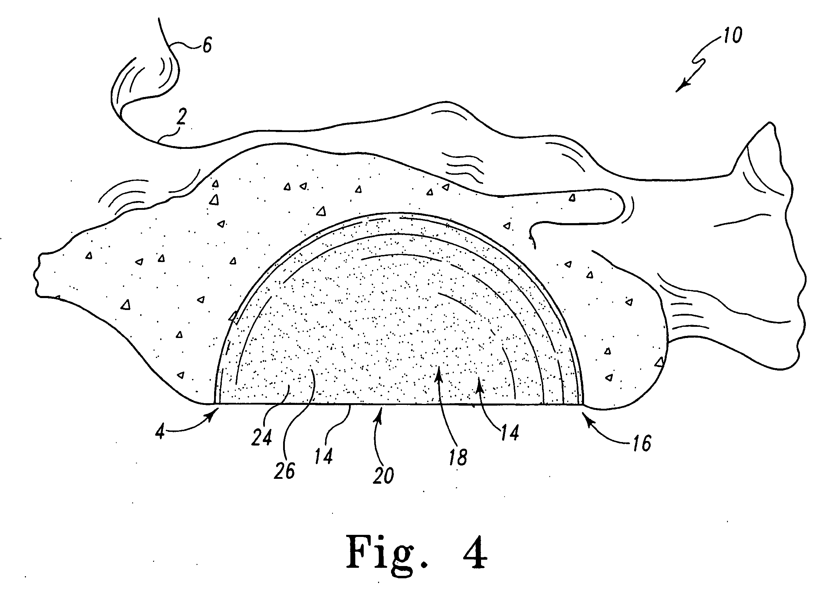

Referring to the drawings, FIGS. 3 and 4 show a prosthetic implant system 10 for securing to bone 2 which can be used to form an articulating joint 12. The prosthetic implant 10 includes a first component 14 having a first portion 16 with a surface 18 adapted to be fixedly secured to the bone 2. At least a portion of the surface 18 of the first portion 16 of the first component 14 has a porous structure. The first component 14 further includes a second portion 20. The second portion 20 may directly provide an articulating surface, or as shown in FIG. 3 the second portion 20 may include a surface 22 for receiving an insert or cup that fits against the second portion 20 and provides the articulating surface. The prosthetic implant 10 further includes a biological component 24 having stem cells 26 at least partially dispersed in a porous structure 19 of the surface 18 of the first portion 16 of the first component 14. The porous structure 19 may be a coating applied to the implant 10 or the implant 10 may be fully porous. For example the implant may be made from a isostatically moulded foam metal or similar biocompatible, metallic load-bearing implant components. The porous structure may be, for example, a sintered bead coating (for example of the kind marketed under the trade mark Porocoat) or a hydroxyapatite (HA) coating. The biological component 24 is dispersed in the porous structure 19 by a device that closely conforms to the component 14. Alternatively, the implant may be a fully porous implant that attaches to bone and may be used to for a scaffold for an implant.

The stem cells 26 of the biological component 24 for placement within the porous structure 19 of the surface 18 of the first portion 16 of the first component 14 may be any stem cell. For example, the stem cells 26 may include mesenchymal stem cells. Mesenchymal stem cells possess the ability to differentiate into a number of cell phenotypes. Within the natural healing cascade, mesenchymal stem cells are influenced by clues from the local environment. Mesenchymal stem cells are activated and maturation is regulated by a series of naturally occurring growth factors that guide them to form osteoblasts, or bone forming cells.

The stem cells 26 for disbursement in the porous structure 19 of the first component 14 may include bone marrow connective tissue progenitor cells. Bone marrow contains two primary cell types. These two types are hematopoietic lineage cells and mesenchymal lineage cells.

Bone marrow is predominantly composed of cells of hematopoietic lineage. These cells play a role in, but are not directly responsible, bone formation. Generally, the surface receptors of these cells bind readily with each other and are less likely to bind to an extra cellular matrix. Mesenchymal lineage cells make up a small fraction of the cells in bone marrow. They include the osteoprogenitor cells, which are directly responsible for bone formation. Osteoprogenitor cells have matrix specific receptors and require attachment to extra cellular matrix for optimal function.

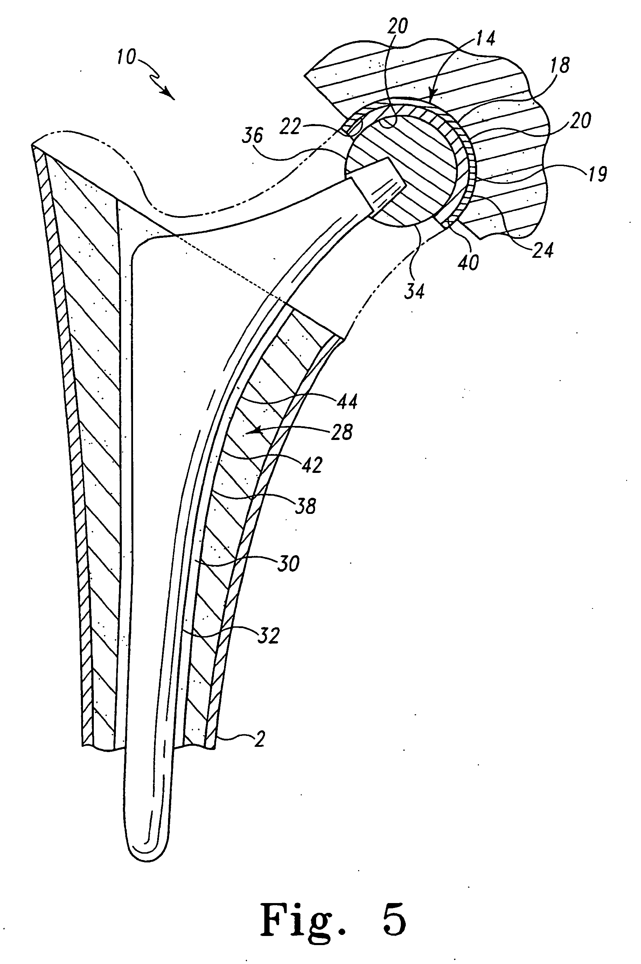

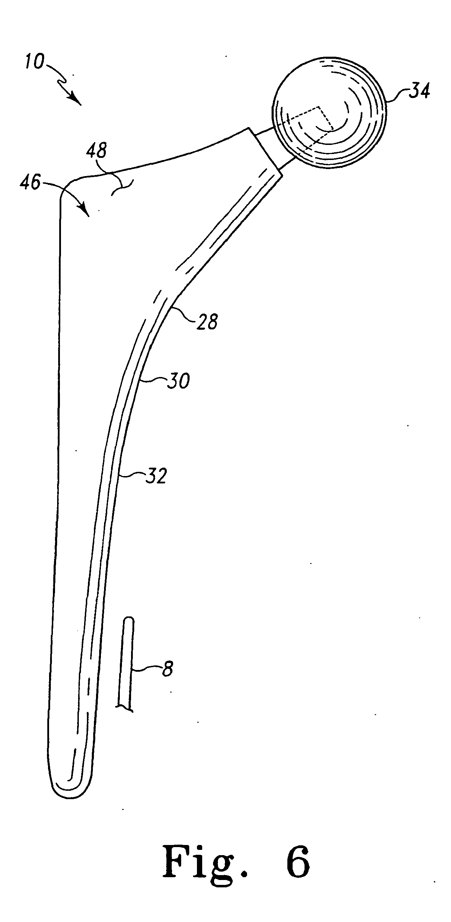

The stem cells 26 for dispersing in the porous structure 19 of the first component 14 may include connective tissue progenitor cells. The prosthetic implant 10 may further include a second component 28 as shown in FIGS. 5 and 6. The second component 18 may include a first portion 30 having a surface 32 adapted to fixedly secure to bone 2 and a second portion 34 adapted to provide a second articulating surface 36 for cooperation with the articulating surface 22 of the second portion 20 of the first component 14.

Referring again to FIG. 6 the implant 10 is shown in position in the acetabulum 4 of hip 2. The first component 14 includes surface 18 which has the porous structure 19. The porous structure 19 is in contact with bone 2 forming the acetabulum 4. The biological compound 24 is dispersed in the porous structure 19 of the surface 18 of the first component 14 and promotes the bony ingrowth into the porous structure 19 to provide for a secure implant 10 in the acetabulum 4 of hip 6.

The implant 10 may be in the form, as shown in FIGS. 5 to 7, of an orthopaedic hip implant. The hip implant 10, as shown in FIG. 5, includes the first portion 30 in the form of distal stem 30 to which head 34 is attached. The head 34 may cooperate directly with first component 14 in the form of a hip cup. The implant 10 may, as shown in FIG. 5, further include a liner 40 which is positioned between the hip cup 14 and the head 34 of the second component or hip stem 28.

The liner 40 may be made of any suitable durable material and may, for example, be made of a polymer, for example ultra high molecular weight polyethylene. The liner 40 may alternatively be made of a metal that is compatible with the human anatomy. It should be appreciated that the surface 18 of the hip cup 14 may include a porous structure 19 to which biological component 24 may be dispersed. It should also be appreciated that surface 32 of distal stem 30 of the hip stem 28 may likewise include a porous structure in the form of porous structure 38. The porous structure 38 may be dispersed with biological compound 42 which includes stem cells 44. The stem cells 44 and biological compound 42 promote bony in growth into the bone porous structure 38 of the surface 32 of the distal stem 30 to provide for a secure hip stem 28 for the implant 10.

Referring now to FIG. 6, the hip stem 28 is shown in greater detail. The hip stem 28 includes distal stem 30 to which head 34 is secured. The distal stem 30 of the hip stem 28 is positioned in cavity 46 formed in medullary canal 48 of femur 8.

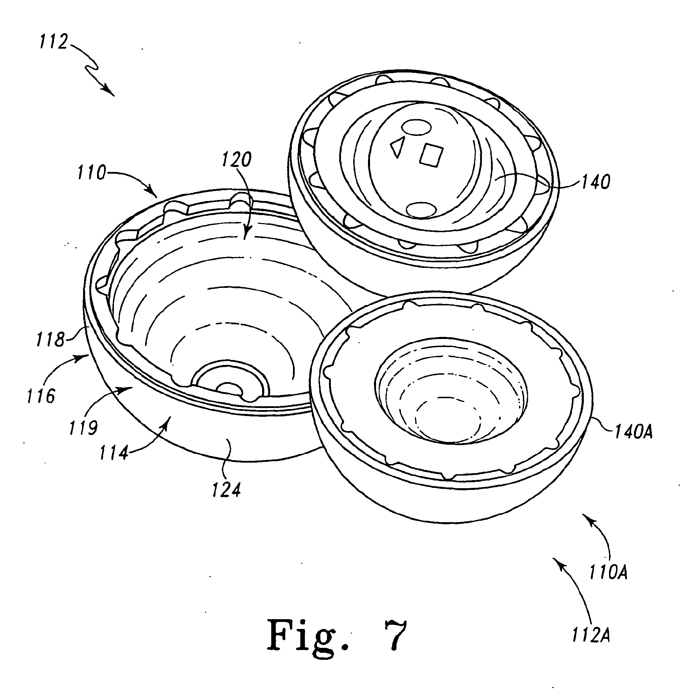

Referring now to FIG. 7, another embodiment of the present invention is shown as implant 110. The implant 110 is very similar to the implant 10 of FIG. 3, but includes a system for providing alternate implant bearing materials for the same shell and head. The implant 110 may be used in an orthopaedic joint 112 and may, as shown in FIG. 7, include a first component 114 in the form of a hip cup or hip shell. The cup 114 includes a first portion 116 including a first surface 118 having a porous structure 119 to receive the biological compound 124. The cup 114 further includes a second portion 120 which may be an articulation surface or may, as shown in FIG. 7, receive a liner or bearing in the form of, for example, polymer liner 140 or alternatively, a ceramic or metallic liner 140A. The ceramic liner 140, together with the cup 114, forms implant 110A which may become part of articulating joint 112A.

While, as shown in FIGS. 3 to 7, the implant of the present invention may be in the form of a total hip replacement orthopaedic joint. It should be appreciated that the implant of the present invention may be an implant for cooperation with other bone. For example, the implant may be utilized for a alternative joint of the body or for use in trauma to assist in repairing bone fracture.

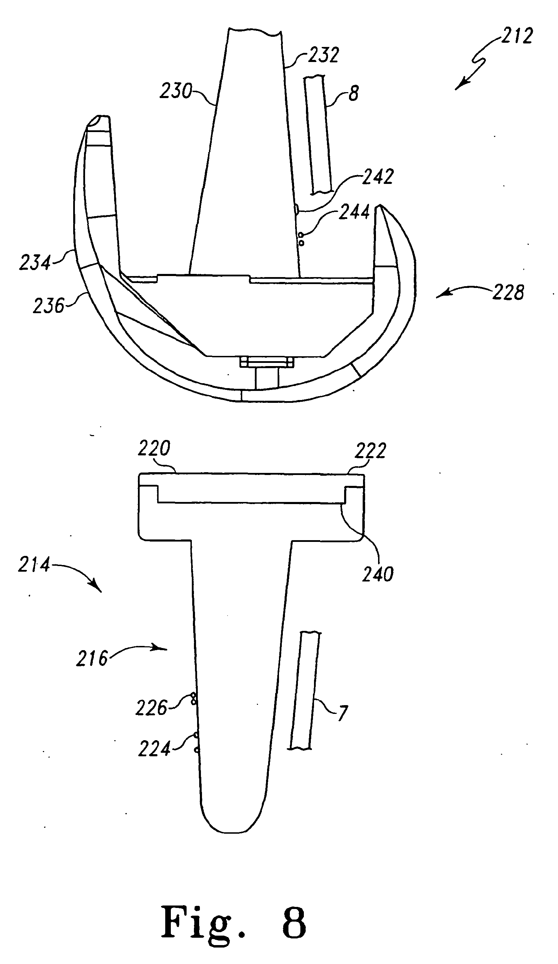

Referring now to FIG. 8, the joint of the present invention may be in the form of, for example, an articulating joint knee prosthesis 212. The knee prosthesis 212 includes a tibial component 214 which cooperates with femoral component 228. The tibial component 214 is fitted into tibia 7 and the tibial component includes a first portion 216 to which a biological compound 214 including stem cells 226 is secured. The tibial component 214 further includes a second portion 220 which receives a liner 240. The liner 240 includes an articulating surface 222 for articulating with the femoral component 228. The implants may be in the form of a total hip, knee, shoulder, ankle, or other joint replacement or partial joint replacement and may, for example, apply to wedges and augments that are used in revision surgeries.

The femoral component 228 includes a first portion 230 in the form of a stem for positioning in the medullary canal of femur 8 of the body. The stem 230 includes surface 232 which receives biological compound 242 including stem cells 244. The femoral component 228 further includes a second portion 234 which defines articulating surface 236 which is in a combination of rolling and sliding contact with articulating surface 222 of the liner 240 of the tibial component 214. It should be appreciated that either the stem 230 of the femoral component or the stem 216 and shoulder 217 of the tibial component 214 may include the biological compound with stem cells or the stem 230 of the femoral component 228 and the stem 216 and shoulder 217 of the tibial component 214 may include the biological compound with stem cells. It should be appreciated that the components that receive the biological compound would need to have corresponding closely conforming fixtures (not shown) which can be placed in the apparatus described herein to pass the biological compound.

It should be also appreciated that the biological compound including stem cells may, after being concentrated and injected into the porous implant matrix with the apparatus of the present disclosure, be utilized for any orthopaedic joint implant which includes a surface in contact with bone. The surface in contact with bone would receive the biological compound. It should be appreciated, for more effective use of the biological compound, the surface to which the biological compound is adhered onto should have a roughened surface and preferably a porous structure. For example, the biological compound and stem cells of the present invention may be utilized on a shoulder prosthesis, an ankle prosthesis, an elbow prosthesis, a wrist prosthesis, or a bone elsewhere in the body adjacent an articulating joint.

The biological compound may be any biological compound which includes stem cells. For example, the biological compound may include bone marrow aspirate. Further the biological compound may include for example granulated calcium phosphate, tri-calcium phosphate or collagen derived bone graft. It should be appreciated the biological compound may include any or up to all three of the calcium phosphate, tri-calcium phosphate and collagen derived bone graft.

The bone marrow aspirate may be processed to provide an enriched population of connective tissue progenitor cells. For example, to provide for the enriched population of connective tissue progenitor cells the bone marrow aspirate may be passed through a porous, biocompatible, implantable graft matrix. Further the biological compound may include growth factors. For example, the growth factors may also include forms of platelet derived growth factors, fibroblast growth factors, epithelial growth factors, transforming growth factor Beta, insulin-like growth factors, parathyroid hormone (PTH) or PTH related peptide, and bone morphoginec proteins.

It should further be appreciated that the biological compound may include other material that may assist in promoting growth of cells. For example, the biological compound may include a material including a collagen and oxidized regenerated cellulose, such as that sold under the trade mark Promogran by Ethicon Inc. The biological compound may either be added to the biological compound that is processed through the porous, biocompatible, implantable graft matrix or be added separately inserted into or adjacent the matrix.

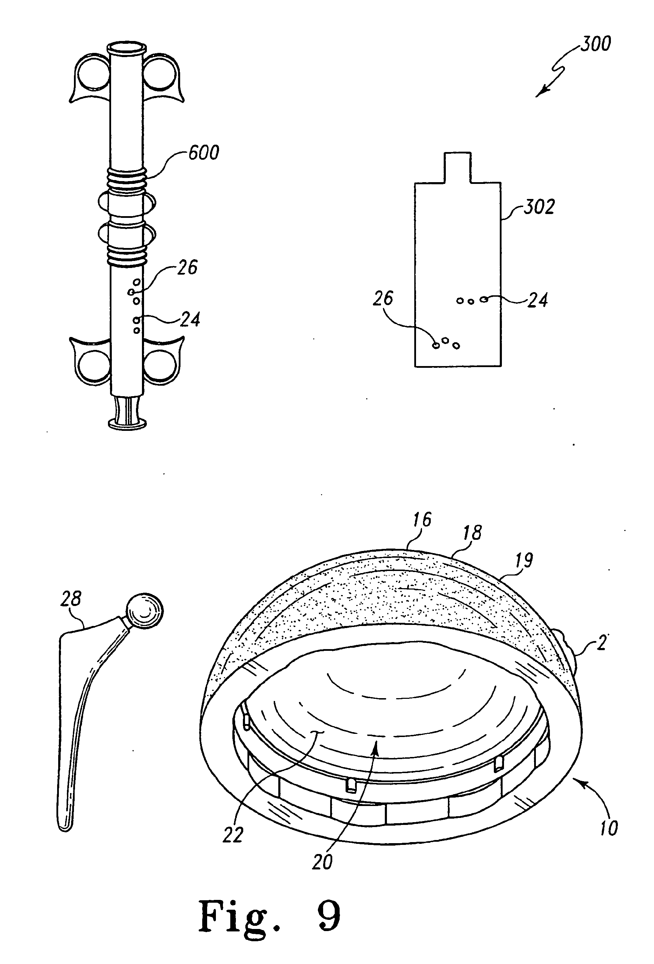

Referring now to FIG. 9, yet another embodiment of the present invention is shown as kit 300. The kit 300 is for use in performing joint arthroplasty. The kit 300 includes, for example, a component, for example a component of an orthopaedic implant. For example, the component may be in the form of hip implant 10 of FIGS. 3 to 7. The implant 10 includes first portion 16 having surface 18 adapted to be fixedly secured to bone 2. At least a portion of the surface 18 of the first portion 16 has porous structure 19. The implant 10 further includes second portion 20 adapted to provide articulating surface 22.

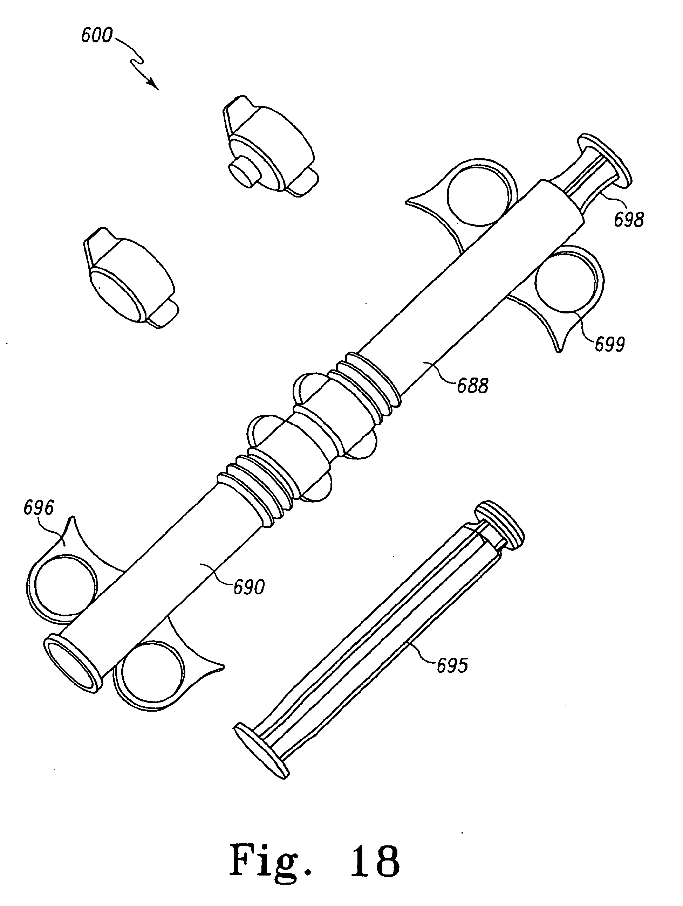

The kit 300 of FIG. 9 further includes biological compound, for example, biological compound 24. The biological compound 24 includes stem cells 26 adapted to be at least partially dispersed in the porous structure 19 of the surface 18 of the first portion 16 of the hip cup implant 10. The biological compound may be utilized in the kit 300 by dispersing the compound on the porous structure 19 of the implant 10 immediately prior to the implantation of the implant 10. The biological compound 24 may be in a fluid form and, as such, may be stored in, for example, vessel 302. The vessel for storing the biological compound 24 may further be utilized to apply the compound 24 to the implant 10. For example, the biological compound 24 may be stored in, for example, instrument 600. The instrument 600 is more fully described in FIG. 18 and in the specification below. The kit 300 of FIG. 9 may further include a second component, for example, hip stem 28 of FIG. 6.

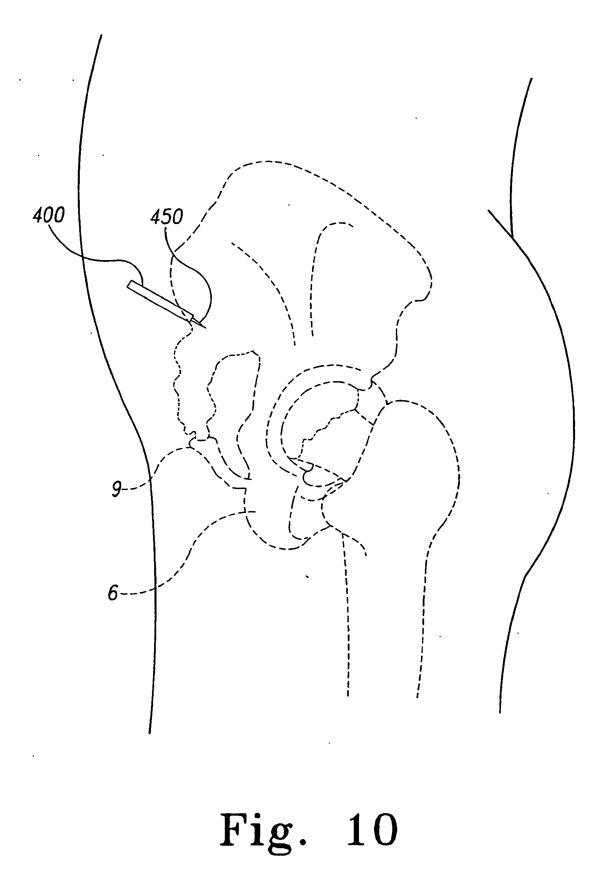

Referring now to FIG. 10, an instrument in the form of syringe 400 is used in extracting the bone marrow aspirate. The syringe 400 includes a needle 450 which may be inserted in iliac crest 9 of the hip 6 of a patient.

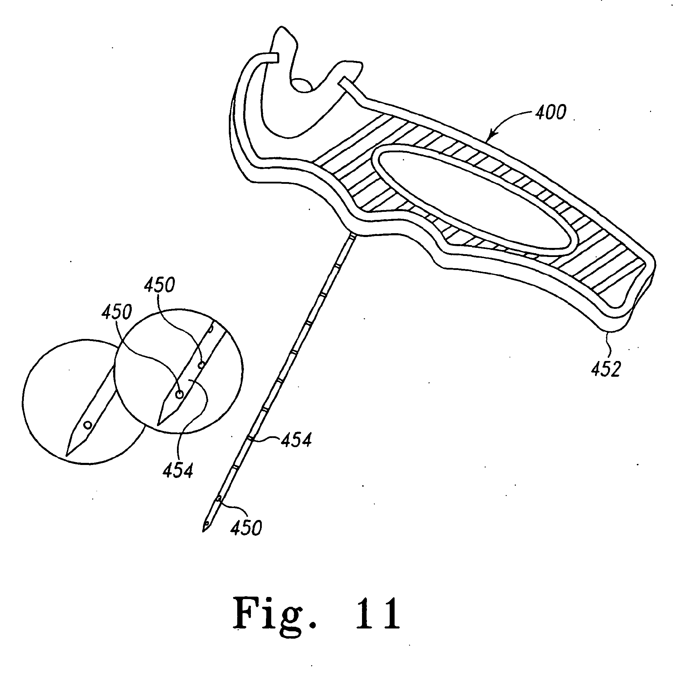

Referring now to FIGS. 10 and 11 the syringe 400 is shown in greater detail. The syringe 400 includes needle 450 which is attached to handle 452. The needle 450 includes openings 454 for receiving the bone marrow aspirate in the iliac crest 9 of the hip 6. The bone marrow aspirate may be a portion of the biological compound 24 in accordance with the present invention.

The method of osteoprogenitor cell selection and concentration which is disclosed in US-6723131, US-6049026, US-5824084 and US-A-2004/0191897 allows the "selective retention" of the important bone forming cells, which can then be combined with banked bone to replicate the components of autogenous bone and be used to join bone. By virtue of its percutaneous minimally invasive approach, this technique eliminates the morbidity associated with open bone graft harvesting. This natural attachment is to a collagen matrix.

The osteoprogenitor cells are harvested by first aspirating bone marrow from the iliac crest with a standard bone biopsy needle, as shown in FIGS. 10 and 11. The bone marrow is then subsequently combined with a specific bone matrix using a patented technology. The processing method builds upon the natural "attachment" tendency of bone-forming cells. Relying on the principle of an affinity column (chemistry, a binding force), the technology facilitates the retention of osteoprogenitor cells within the matrix, while discouraging retention of other non-bone forming cells.

A method of providing composite bone marrow graft material having an enriched population of progenitor cells generally comprises the following steps: 1. obtaining a bone marrow aspirate; 2. contacting the bone marrow aspirate with a porous biocompatible implantable matrix (e.g. by flowing the aspirate through the matrix) to provide a progenitor cell-enriched matrix having an enriched population of progenitor cells; 3. mechanically mixing the enriched matrix to provide substantially uniform progenitor cell distribution throughout; and 4. draining the matrix of excess liquid. Composite bone marrow graft material thus prepared is then implantable into a patient or patient, and is effective to induce bone healing and/or bone regeneration.

Preferably, the method includes the step of adding clot material to the enriched matrix. The adding of clot material may confer handling characteristics on the graft material for a short period of time such that the surgeon can use it in bone grafts more easily. When using the graft material in metallic structures a clotting agent may not be needed since the implant will still be easy for the surgeon to handle even after the seeding process.

The steps of a method as outlined above comprise several functional elements which will now be described. Such functional elements include a bone marrow aspirate, a porous biocompatible implantable matrix, and preferably clot material. Following a description of these functional elements is a description of the preferred methods and apparatus for preparing a composite bone graft of the present invention.

Bone marrow aspirate contains plasma, nucleated progenitor cells (progenitor cells), nucleated hematopoietic cells, endothelial cells, and cells derived from peripheral blood, including red cells and platelets. Because bone marrow aspirate contains peripheral blood, it is preferred that the aspirate be collected in a syringe containing an anticoagulant. Suitable anticoagulants include heparin, sodium citrate, and EDTA. Preferably, a bone marrow aspirate for use in a method of the present invention is obtained from the patient who will receive the graft (the patient). Less preferably, the bone marrow aspirate can be obtained from another immunologically compatible donor.

The matrix comprises a porous, biocompatible, implantable matrix. Preferably, the matrix has a bioactive surface. Examples of porous biocompatible, implantable graft matrix materials having a bioactive surface include ceramics comprising calcium phosphate such as hydroxyapatite or tri-calcium phosphate, as well as demineralized or mineralized bone matrix. Other suitable matrix materials include biopolymers such as polylactic acid, polyglycolic acid, polygalactic acid, polycaprolactone, polyethylene oxide, polypropylene oxide, polysulphone, polyethylene, and polypropylene. Still other suitable matrix materials are hyaluronic acid, which may be purified with or without crosslinking, bioglass and collagen.

More preferably, cell adhesion molecules are bound to the surface of the matrix substrate. The term "cell adhesion molecules" includes laminins, fibronectin, vitronectin, vascular cell adhesion molecules (V-CAM), intercellular adhesion molecules (I-CAM), tenascin, thrombospondin, osteonectin, osteopontin, bone sialoprotein, collagens, or any other molecules or components effective to promote selective adhesion of progenitor cells to the substrate surface. Some of the above cell adhesion molecules have been found to positively affect early adhesion of mescenchymal stem cells given short direct surface exposure times. Mescenchymal stem cells in some embodiments of the present invention may be constantly flowing across the surface, whether that surface is treated or not. Molecules that promote adhesion of these specific mescenchymal stem cells in spite of brief exposure to the cells are sought.

Preferably, the matrix has sufficient porosity to yield an increase in total matrix surface area available for progenitor cell-adhesion relative to a nonporous solid having identical external dimensions with greater increases being preferred. Such an increase may be, for example, an increase that is at least a 2-fold, 3-fold, 5-fold, 7-fold, or a 10-fold, increase. Such an increase in total surface area can be achieved by using a matrix substrate comprising powder, granules, fibres, some combination thereof, or a single highly porous substrate mass. The matrix substrate may be bone marrow aspirate and may alternatively be a combination of bone marrow aspirate and granulated calcium phosphate, tri-calcium phosphate or collagen -derived bone graft, with optional inclusion of a known anti-infective or anti-microbial or growth factor agent. Preferably, the size of the pores in the matrix for progenitor cell-adhesion is greater that 20 µm with larger pore sizes being preferred. For example the pore size may be, 50, 100, 500, or 1000 µm, in order to facilitate penetration of progenitor cells through the pore openings into the void volume of the matrix material, thereby availing of the additional surface area within the pores. These particles should be sized to be dispersed in the porous structure of most porous coated prostheses for use in joint arthroplasty. Generally these particles should be reduced to a size of less than 1000 µm, with smaller sizes preferred, such particles may be used with method and apparatus of the present invention.

Particularly suitable matrix materials include isolated mineralized cancellous bone sections, powders or granules of mineralized bone, demineralized cancellous bone sections, powders or granules of demineralized bone, guanidine-HCl extracted demineralized bone matrix, sintered cortical or cancellous bone, coralline hydroxyapatite sold by Interpore International Inc of 181 Technology Drive, Irvine, California 92618, under the trade marks Interpore 500, or Interpore 200, granular ceramics such as that incorporated into the bone graft substitute Collagraft sold by Zimmer, granular or block ceramics such as that incorporated into the graft substitute Vitoss sold by Orthovita Inc of 45 Great Valley Parkway, Malvern, Pennsylvania 19355, and filamentous sponges such as those made from collagen.

A preferred matrix is prepared as a combination of particulate bone material and fibrous bone material. The particulate bone material is preferably derived from spongy human bone, preferably cancellous bone, for example, from a distal end of long human bones. The fibrous bone material is preferably derived from cortical bone. Both the particulate and the fibrous bone materials can be obtained from a bone bank, or optionally from the patient. When obtained from the patient, the bone material is manipulated intraoperatively in the operating room to conform to the desired particulate and fibrous characteristics via known bone manipulation means.

The particulate bone material is provided as allograft cancellous bone particles in the form of chunks, chips or fragments, preferably having a mean diameter of at least 1 mm, more preferably at least 2 mm. Preferably the mean diameter of the particles is not more than 15 mm, more preferably not more than 8 mm. Most preferably, the fibrous bone material is provided as allograft demineralized cortical bone fibres of at least 5 mm, more preferably at least 1 cm, more preferably at least 2 cm, more preferably at least 3 cm, more preferably at least 4 cm, and most preferably at least 5 cm, in length. Optionally the fibrous bone material is provided as a mixture of fibres of varying lengths, preferably at least 5 mm. Preferably, the varying lengths are not more than about 15 cm, more preferably not more than 5 cm, 4 cm, 3 cm or 2 cm. Optionally, the fibrous bone material is supplied as a flexible mat, for example of the type sold under the trade mark Grafton Flex by Osteotech Inc of 51 James Way, Eatontown, New Jersey, 07724. The particulate bone material may be bone marrow aspirate and may alternatively be a combination of bone marrow aspirate and granulated calcium phosphate, tri-calcium phosphate or collagen -derived bone graft, with optional inclusion of a known anti-infective or anti-microbial or growth factor agent. These particles are much too large to be dispersed in the porous structure of most porous coated prostheses for use in joint arthroplasty. When these particles can be reduced to a size of less than 1000 µm, such particles may be used with apparatus of the present invention.

The particulate and fibrous bone materials are combined to form a preferred composite matrix in the following manner. Bone fibres, preferably demineralized cortical bone fibres having lengths as described above, are combined with particulate bone particles in the following preferred proportion: about 225, less preferably 200 to 300, less preferably 150 to 375, less preferably 100 to 450, less preferably 75 to 500, less preferably 25 to 1000 mg dry weight of demineralized cortical bone fibres, with about 10, less preferably 8 to 12, less preferably 6 to 14, less preferably 4 to 16, less preferably 2 to 18, less preferably 1 to 25 cm³ (bulk volume) of particulate bone particles having a mean diameter of 1 to 15, preferably 2 to 8 mm. These particles are much too large to be dispersed in the porous structure of most porous coated prostheses for use in joint arthroplasty. When these particles can be reduced to a size of less than 1000 µm, such particles may be used with the apparatus of the present invention.

Optionally, demineralized cortical bone fibres can be obtained from a flexible mat comprising such fibres. When such a mat is used, it is first washed free of any toxic or hyperosmolar material that may be present, such as glycerol, using an isotonic solution. The mat is then suspended in saline, or other suitable isotonic solution, to facilitate separation of the individual bone fibres. The separated bone fibres are combined with particulate bone material in the following proportion to form a preferred composite matrix: the fibres from one mat having initial dimensions of 2.5 cm × 5 cm × 2.5 mm (initial volume of about 3.1 cm³) with about 10 cm³, less preferably 8 to 12 cm³, less preferably 6 to 14 cm³, less preferably 4 to 16 cm³, (bulk volume) of particulate bone particles having a mean diameter of 1 to 15, preferably 2 to 8 mm. The mat may be bone marrow aspirate and may alternatively be a combination of bone marrow aspirate and granulated calcium phosphate, tri-calcium phosphate or collagen -derived bone graft, with optional inclusion of a known anti-infective or anti-microbial or growth factor agent. These particles are much too large to be dispersed in the porous structure of most porous coated prostheses for use in joint arthroplasty. When these particles can be reduced to a size of less than 1000 µm, such particles may be used with the apparatus of the present invention.

It should be noted that when grafts of differing size are necessary, a composite matrix of different size can be prepared to conform with the above-stated proportion of fibrous to particulate bone according to the present invention. For example, (assuming uniform bulk density) 20 cm³ of particulate bone can be combined with 450 mg of bone fibres to provide a preferred composite matrix. These particles are much too large to be dispersed in the porous structure of most porous coated prostheses for use in joint arthroplasty. When these particles can be reduced to a size of less than 1000 µm, such particles may be used with the apparatus of the present invention..

It is believed that inclusion of a bone marrow clot may improve the efficacy of a composite bone graft for one or several of the following reasons. First, it is possible that some cells important to the process of successful bone healing do not attach to the graft matrix and therefore are not sufficiently concentrated in (or possibly are even excluded from) the graft site, resulting in ineffective or inefficient healing at that site. The polymerization of fibrinogen into fibrin resulting from the clotting cascade (further explained below) may provide a valuable supplemental matrix promoting the attachment and migration of cells important to the healing response at the graft site. Such cells include migratory endothelial cells which proliferate to form tubular structures that are important precursors to the formation of blood vessels via angiogenesis.

A second possibility is that the physiologic process of forming a clot at the graft site creates an improved environment for transplanted osteogenic cells at that site. Specifically, clotting of the non-anticoagulated bone marrow aspirate results in the activation of platelets contained therein, resulting in platelet degranulation. Platelet degranulation in turn releases growth factors and osteotropic cytokines which might otherwise be absent from the graft site. Several important bioactive factors released during this process include platelet derived growth factor (PDGF), epidermal growth factor (EGF), fibroblast growth factors (FGFs), and transforming growth factor beta (TGF-beta)

In addition, fibrin matrix formed from fibrinogen as a result of the clotting cascade may provide important stability at the graft site during the immediate post-operative period. Furthermore, the process of fibrinolytic activity that occurs over the first several days following graft implantation provides an additional source for angiogenic factors (e.g. fibrin split products as known in the art) during the early stages of graft incorporation. It is believed that the resulting angiogenesis at the graft site following implantation may enhance the formation of new blood vessels in the site providing a source of nourishment for the freshly implanted progenitor cells and other cells responsible for bone healing and growth, thus accelerating the healing response.

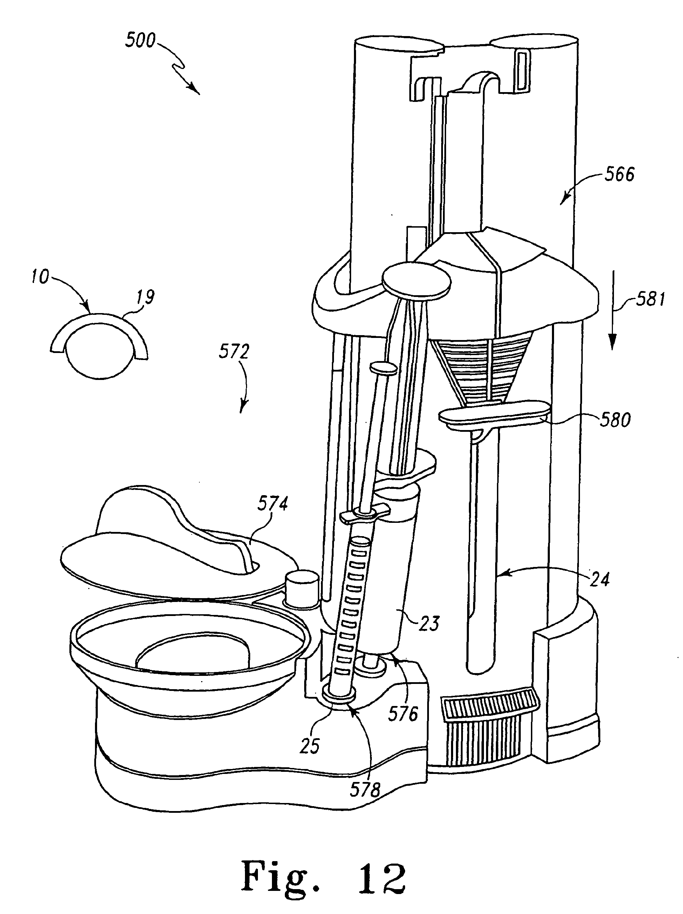

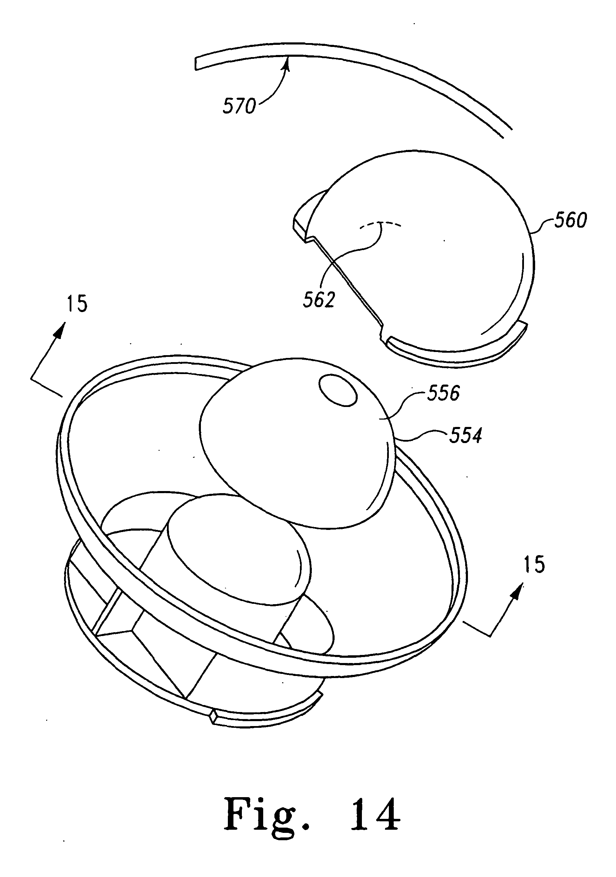

FIGS. 12 to 15 show an instrument 500 which is for use in applying a biological compound, for example biological compound 24 including stem cells 26 to the porous surface for example porous surface 19 of a prosthetic implant for example prosthetic implant 10 of FIGS. 2 to 6. The prosthetic implant 10 may be implanted into a surface of a bone too for example at acetabulum 4 of a hip 6.

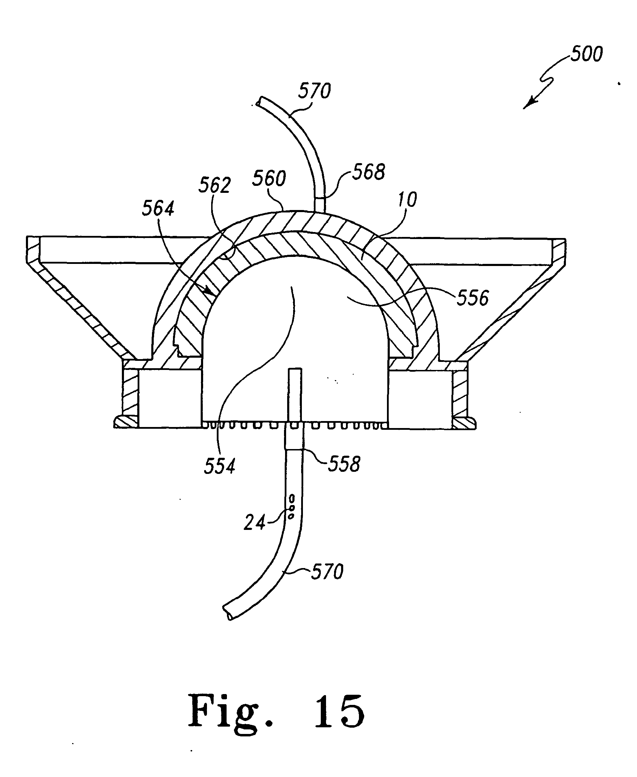

Referring now to FIG. 15, the instrument 500 includes a first member 554 having a first surface 556. The instrument further includes a connector 558 for receiving the biological compound 24. The connector 558 may be connected to a portion of the first member 554. The instrument 500 further includes a second member 560 which can be connected to the first member 554. The second member 560 includes a first surface 562. The first surface 556 of the first member 554 and the first surface 562 of the second member 560 define a cavity 564 positioned between the first member 554 and the second member 560. The prosthetic implant 10 can be positioned in the cavity 564. The instrument 500 may further include a second connector 568 connected to, or being a part of, the second member 562 the second connector 568 may be connected to conduit 570 advancing and circulating the biological compound 24. The conduit 570 is likewise connected to connector 558.

Referring to FIG. 12 to 13, the instrument 500 further includes a device 566 for containing a quantity of the biological component 24. The device 566 can be connected to the first connector 558 of the first member 554. The device 566 is adapted to permit at least a portion of the biological component 24 to pass from the device 566 and into the cavity 564.

Referring now to FIG. 12 the instrument 500 is shown with the device 566 having a loading station 572 for receiving the implant 10. The loading station 572 may optionally include a cover 574 for covering the implant 10 after the implant 10 is positioned in loading station 572. The instrument 500 may further include a first port 576 for receiving bone marrow aspirate 23, as well as, a second port 578 for receiving anti-infection and anti-bacterial component compound 25. The device 566 of the instrument 500 may further include a plunger or handle 580 which when moved in the direction of arrow 581 causes the biological component 24 to circulate through the device 566.

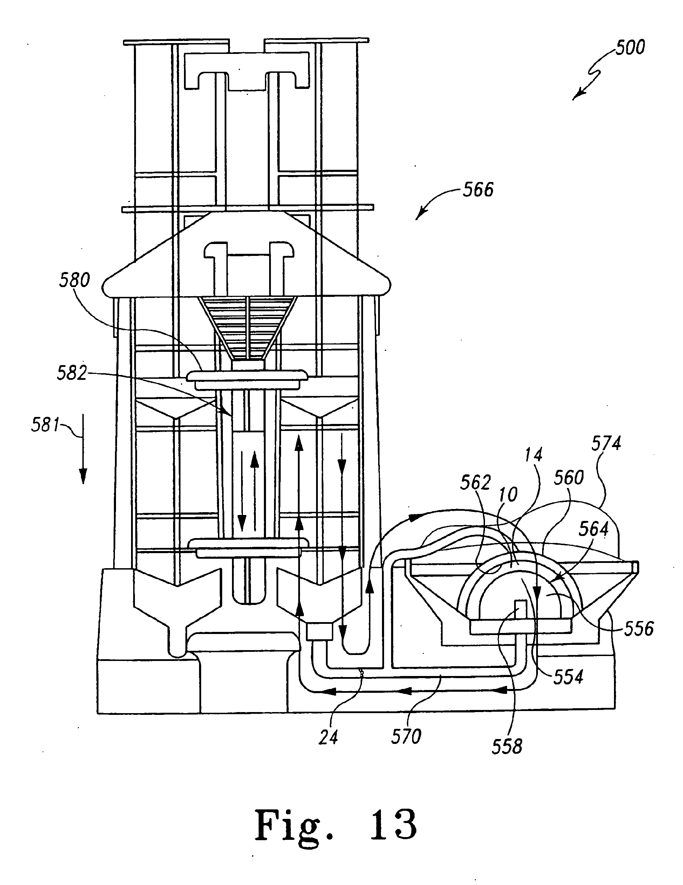

Referring now to FIG. 13, the instrument 500 is shown with the device 566 as well as first member 554 and second member 560. The first member 554 is adapted to receive implant 510 when cover 574 is raised. The implant 10 in the form of a hip cup is placed with the concave surface of the cup 10 positioned downwardly. The second member 560 is positioned over the implant 10 with the first member 554 and the second member 560 defining the cavity 564 there between. The conduit 570 circulates material from second member 560 through the porous surface of implant 10 and into and past first member 554 and through conduit 570 utilizing, for example, pump 582. The pump 582 is activated by plunger or handle 580 in the direction of arrow 581.

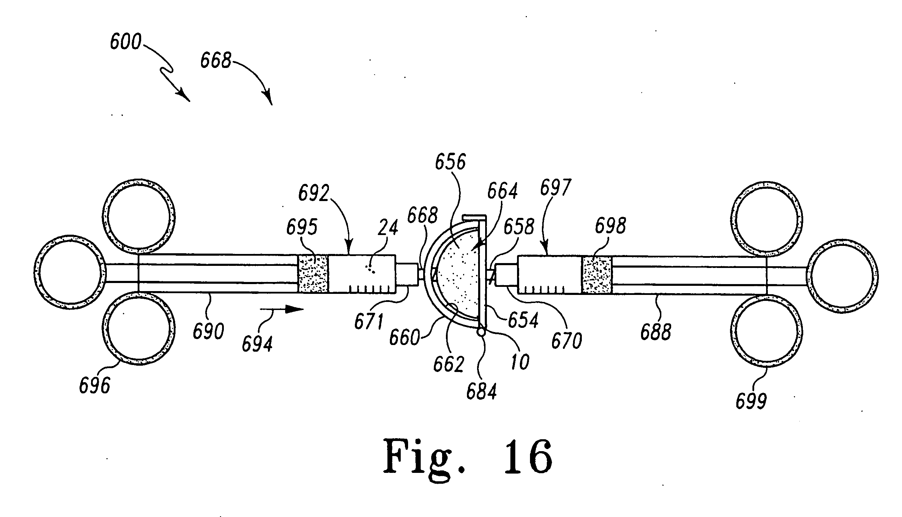

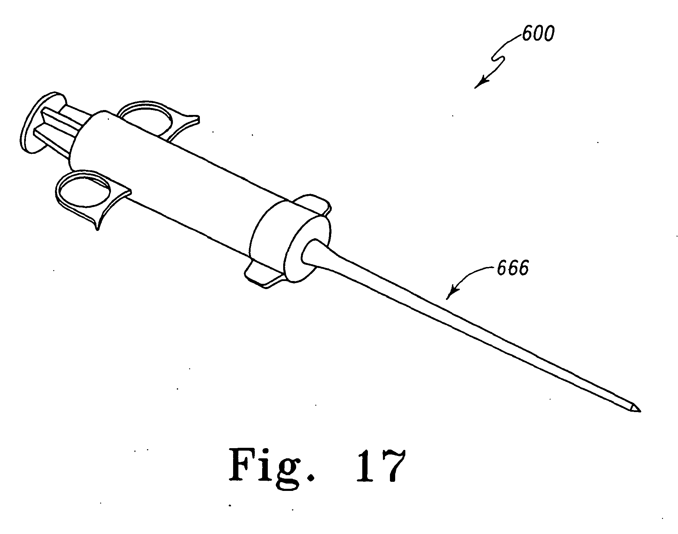

FIGS. 16 to 20 show an instrument 600 which can perform a similar function to that of the instrument 500 described above with reference to FIGS. 12 to 15, except that the instrument 600 is simpler and less expensive. The instrument 600 includes a first member 654 similar to the first member 554 of the instrument 500 of FIGS. 12 to 15. The instrument 600 further includes a second member 660 similar to the second member 560 of the instrument of FIGS. 12 to 15.

The first member 654 includes a first surface 656 for cooperation with the implant 10. The first member 654 further includes first connector 658 for receiving conduit 670 in the form of first needle.

The second member 660 includes a first surface 662 for cooperation with prosthetic component 10. The second member 660 may include a second connector 668 for cooperation with second needle 671.

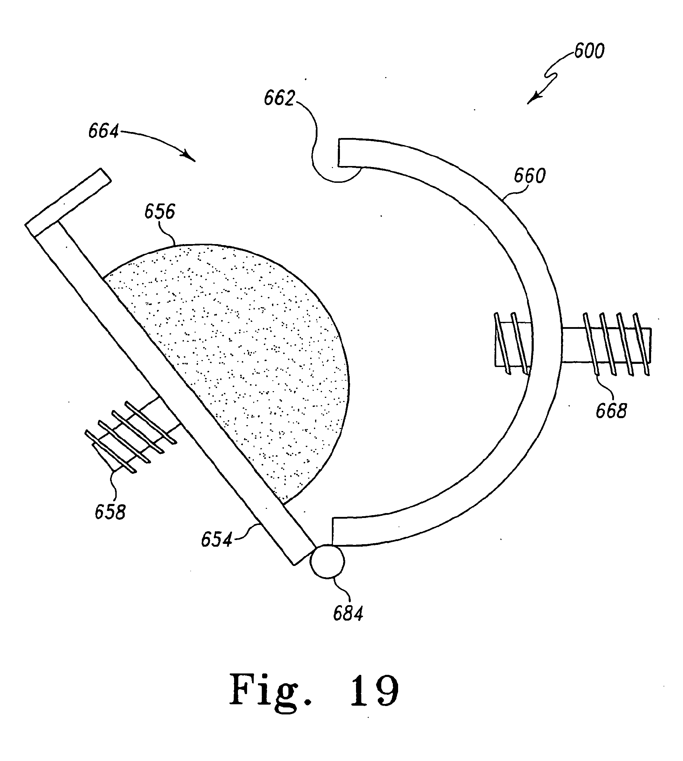

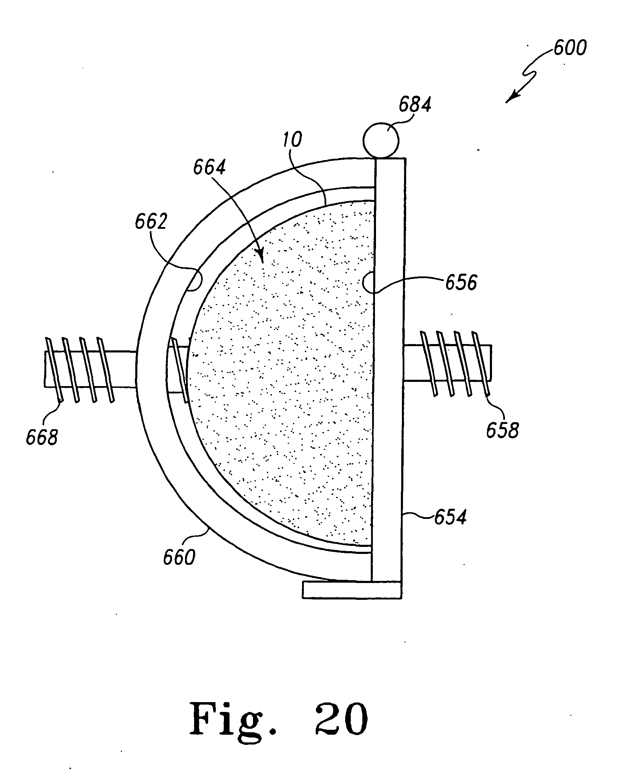

The first member 654 may cooperate with the second member 660 in any suitable fashion such that cavity 664 is formed there between. For simplicity, and as shown in FIGS. 19 and 20, the first member 654 and the second member 660 form a hinge 684 positioned there between. The hinge 684 permits the first member 654 to pivot with regard to second members 660 to permit the implant 10 to be loaded and unloaded into the instrument 600.

Referring again to FIG. 16, the device 666 of the instrument 600 may as shown in FIG. 16 be in the form of a first syringe 688 and a second syringe 690. The first syringe 688 or the second syringe 690 may be filled with the biological compound 24. The other syringe may, for example, be filled with anti-infection, anti-bacterial, or another compound.

For example and as shown in FIG. 16, the first syringe 688 includes a first syringe connector 670 which receives first connection 658 of first member 654. Similarly second syringe 690 includes second syringe connector 671 which receives second connector 668 of second member 660.

Biological compound 24 is loaded into, for example, cavity 692 formed in second syringe 690. The biological compound 24 is advanced in the direction of arrow 694 by advancing second plunger 695 in the direction of arrow 694 by pushing with second handles 696. The compound 24 advances through second needle 671 and through second connector 668. Next, the biological compound 24 advances into cavity 664, through first connector 658, through first syringe connector 670 and into cavity 697 formed in first syringe 688.

Once the biological material 24 has filled the cavity 697 formed in first syringe 688, the material 24 is returned to second syringe 690. This material 24 is retuned by advancing the material 24 with the first plunger 698 of first syringe 688 in a direction opposed to arrow 694 by pushing with first handle 699 of first syringe 688.

By advancing the plungers back and forth, the biological compound 24 may be passed through multiple times through the component 10 to cause the fluid to disperse in the porous surface of the orthopaedic implant.

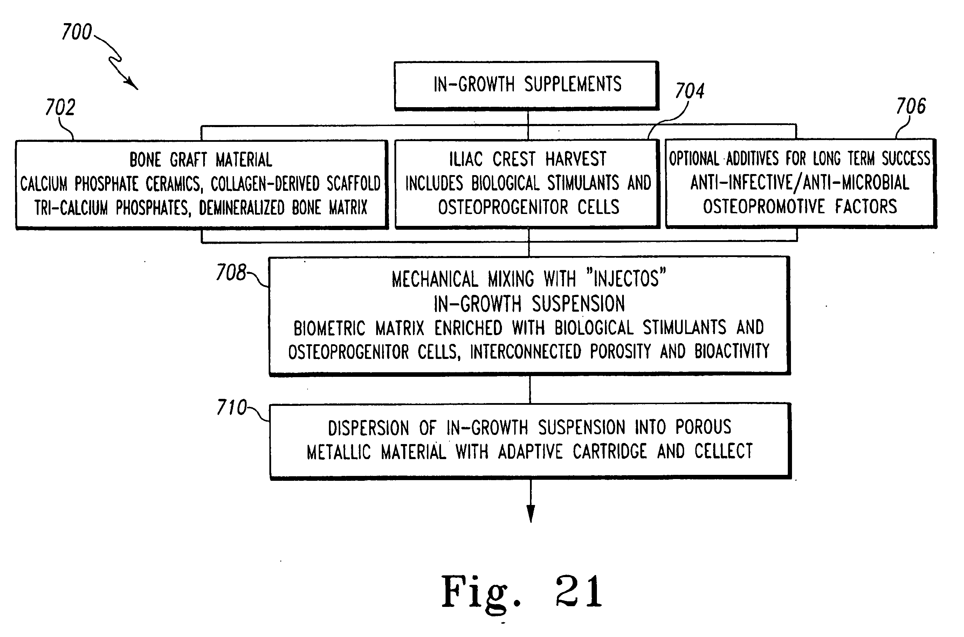

A method 700 of using apparatus according to the invention is illustrated in FIG. 21. The method 700 includes a step 702 of providing bone graft material including for example calcium phosphate, ceramics, collagen derived scaffolding, tri-calcium phosphates, and demineralized bone matrix. The method 700 further includes step 704 of performing an iliac pressed harvest which includes biological stimulants and osteoprogenitor cells. The method 700 may further include step 706 of adding additives for long term success such as anti-infective or anti-microbial osteopromitive factors. The method 700 further includes step 708 of mechanically mixing with additional material including an ingrowth suspension. The biomemenic matrix enriched with biological stimulants and osteoprogenitor cells include interconnective porosity and bioactivity. The method further includes step 710 of dispersing the in-growth suspension into porous metallic material with adaptive cartridge and instruments of the present invention.

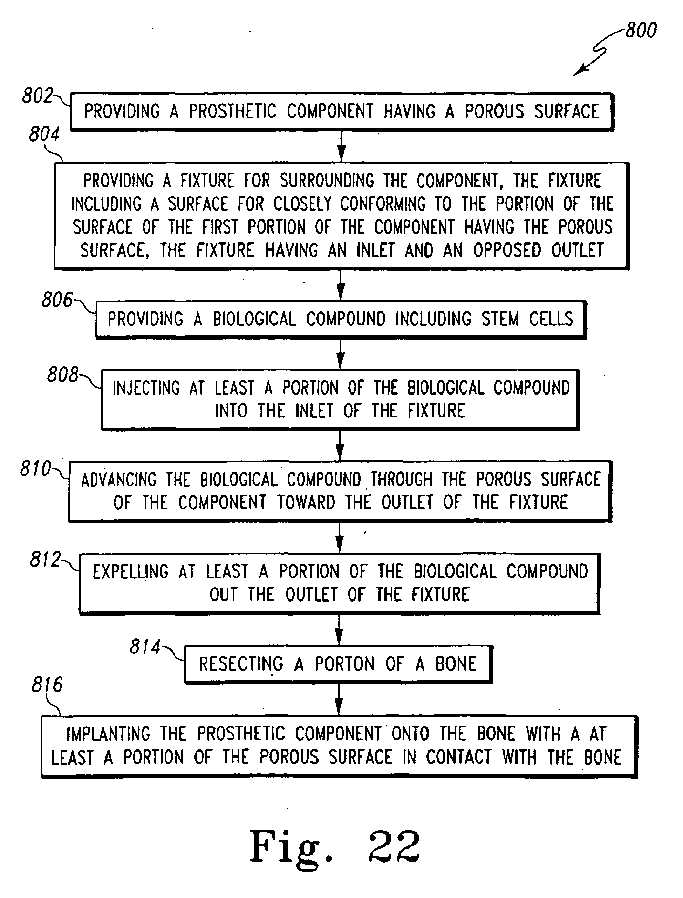

A method 800 of providing joint arthroplasty according to the invention is illustrated in FIG. 22. The method 800 includes a step 802 of providing a prosthetic component having a porous surface and a step 804 of providing a fixture for surrounding the component. The fixture includes a surface for closely conforming to the portion of the surface of the first portion of the component having the porous surface. The fixture has an inlet and an opposed outlet.

The method 800 also includes a step 806 of providing a biological compound including stem cells and a step 808 of injecting at least a portion of the biological compound into the inlet of the fixture. The method 800 also includes a step 810 of advancing the biological compound through the porous surface of the component toward the outlet of the fixture and a step 812 of expelling at least a portion of the biological compound out the outlet of the fixture. The method 800 also includes a step 814 of resecting a portion of a bone and a step 816 of implanting the prosthetic component onto the bone with a at least a portion of the porous surface in contact with the bone.

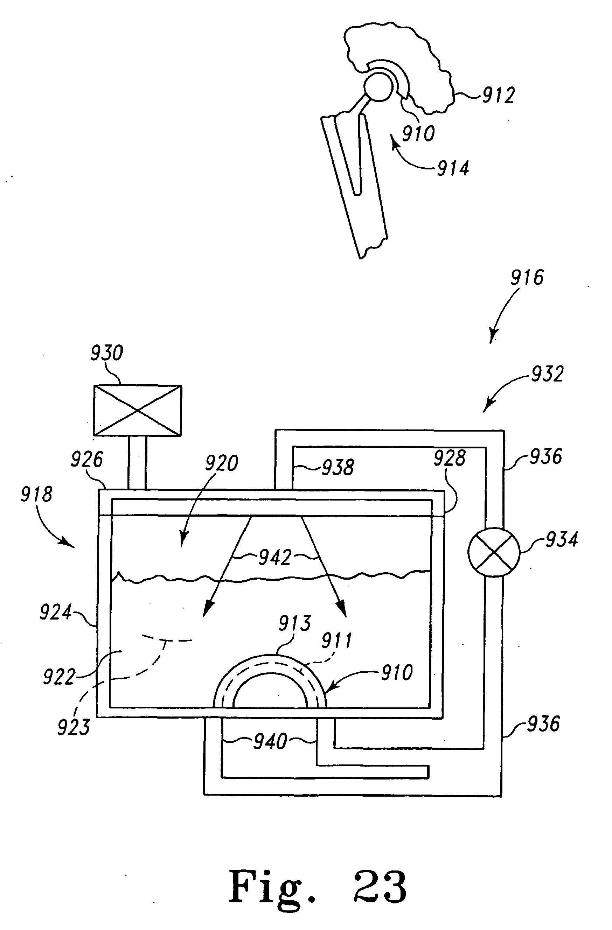

FIG. 23 shows a prosthetic implant 910 for use in for securing to bone 912 to form an articulating joint 914. The implant 910 may be in the form of a component 911 with a porous surface 913. The porous surface 913 may be, for example, in the form of a hydroxyapatite coating or a sintered particle coating. Alternatively the component 911 may be fully porous with the porous surface 913 being a portion of the fully porous component 911. For example, the fully porous component 911 may be an isostatically moulded foam metal or similar biocompatible, metallic load-bearing implant component. The implant 910 may be for a total hip, knee joint replacement or a component for a partial joint replacement. The implant 910 may, for example, apply to component such as wedges and augments that are used in revision surgeries. As shown in FIG. 23 the implant 910 is a hip cup.

As shown in FIG. 23 the implant 910 may be prepared in an apparatus 916. The apparatus 916 includes a container 918 defining a cavity 920 for receiving the implant 910. The container 918 is also adapted for containing a biological compound 922 including stem cells 923. The container 918 may have any shape and be made of any materials capable of receiving the implant 910 and containing the biological compound 922. The container 918, may, as shown, include a base 924 and a lid 926. A seal 928 may be used to secure the base 924 to the lid 926 and to permit the cavity 920 to maintain a pressure above ambient pressure. The apparatus 916 includes a pressurizing device 930 in the form of a pump to provide the elevated pressure within the cavity 920. A biological compound circulation system 932 may include a fluid pump 934 and conduits 936 to circulate the biological compound 922 through the cavity 920.

The implant 910 as shown in FIG. 23 may be prepared by a process which includes the steps of preparing the component 911 with the porous surface 913 to form a prosthetic implant 910. The steps include placing the prosthetic implant 910 including the porous surface 913 in the cavity 920 of the container 918 and directing the biological compound 922 including stem cells 923 into the cavity 920 of the container 918. The steps also include submersing the porous surface 913 of the implant 910 with the biological compound 922 and sealing the container 920 with the implant 910 submersed in the biological compound 922. The steps also include submitting the cavity 920 of the container 918 to a pressure greater than ambient pressure to urge the biological compound 922 into the porous surface 913 of the prosthetic implant 910.

The step of preparing the component 911may include the step of preparing the component 911 with a fully porous structure, the fully porous structure including the porous surface 913.

The process may further include a step of circulating the biological compound 922 with the circulation system 932 including the fluid pump 934 and the conduits 936. The circulation may enhance the adherence of the biological compound 922 to the porous surface 913 of the component 911. The conduits 936 may be positioned such that the conduits include an inlet 938 and an outlet 940 to that may be strategically positioned to direct the biological compound 922 to flow in the direction of arrows 942 through the porous surface 913 of the component 911.

The step of submitting the cavity 920 of the container 918 to a pressure greater than ambient pressure may include submitting the cavity 920 to a pressure greater than 0.101 MPa (1 atmosphere), preferably less than 0.507 MPa (5 atmosphere).

The step of preparing the component 911 with a porous surface 913 may include the step of preparing the component 911 with a hydroxyapatite coating.

While the adherence of the biological compound 922 to the porous surface 913 of the component 911 is enhanced by the application of pressure in the container 918, it should be appreciated that biological compound 922 may adhere to the porous surface 913 of the component 911 to form the implant 910 in the absence of pressure in the container 918 above ambient pressure.

## Claims

1. A kit for use in preparing a component of a joint prosthesis for use in joint : arthroplasty, in which the kit comprises:
a component (10) of a joint prosthesis having porous surface portion (18) formed from a metallic material, the porous surface portion having a porous bone engaging surface for securing to a bone to form an articulating joint;
a biological compound (24) including stem cells (26) adapted to be at least partially dispersed in the porous structure of the bone engaging surface of the porous surface portion;
a vessel (566) for containing the biological compound; and
a fixture (544) for receiving the component so that it is surrounded by the fixture, the fixture including a surface (562) which closely conforms to the surface of the porous portion of the component, the fixture having an inlet (570) and an opposed outlet (558); and
a device (582) for advancing the biological compound from the vessel into the inlet of the fixture so that it is injected into the porous portion of the component, and so that excess biological compound is advanced out of the outlet of the fixture.

2. The kit of claim 1, in which the stem cells comprise at least one of mesenchymal stem cells, bone marrow connective tissue progenitor cells, and connective tissue progenitor cells.

3. The kit of claim 1, in which the joint prosthesis component has an articulating portion which, when the component is secured to a bone, (a) provides an articulating surface or (b) can receive a further component which provides an articulating surface.

4. The kit of claim 3, which includes a second component including a bone engaging portion having a surface adapted to be fixedly secured to bone and an articulating portion which can provide an articulating surface for cooperation with the articulating surface of the articulating portion provided by the said joint prosthesis component.

5. The kit of claim 1, in which the joint prosthesis component is an acetabular cup component of a hip joint prosthesis.

## Patentansprüche

1. Kit zur Verwendung bei der Herstellung einer Komponente einer Gelenkprothese zur Verwendung bei einer Gelenk-Arthroplastik, wobei das Kit umfasst:
eine Komponente (10) einer Gelenkprothese mit porösem Flächenabschnitt (18), der aus einem metallischen Material gebildet ist, wobei der poröse Flächenabschnitt eine poröse Knochenanlagefläche zur Sicherung an einem Knochen aufweist, um eine Gelenkverbindung zu bilden;
eine biologische Zusammensetzung (24), die Stammzellen (26) enthält, die eingerichtet sind, um in der porösen Struktur der Knochenanlagefläche des porösen Flächenabschnitts zumindest teilweise verteilt zu werden;
einen Behälter (566) zur Aufnahme der biologischen Zusammensetzung; und
eine Halteeinrichtung (544) zur Aufnahme der Komponente, so dass sie von der Halteeinrichtung umgeben ist, wobei die Halteeinrichtung eine Fläche (562) enthält, die sich nahe an die Fläche des porösen Abschnitts der Komponente anschmiegt, wobei die Halteeinrichtung einen Einlass (570) und einen gegenüberliegenden Auslass (558) aufweist; und
eine Einrichtung (582) zum Fördern der biologischen Zusammensetzung aus dem Behälter in den Einlass der Halteeinrichtung, so dass sie in den porösen Abschnitt der Komponente injiziert wird und so dass überschüssige biologische Zusammensetzung aus dem Auslass der Halteeinrichtung gefördert wird.

2. Kit nach Anspruch 1, wobei die Stammzellen mesenchymale Stammzellen und/oder Bindegewebevorläuferzellen aus Knochenmark und/oder Bindegewebevorläuferzellen umfassen.

3. Kit nach Anspruch 1, wobei die Gelenkprothesenkomponente einen Gelenkabschnitt aufweist, der, wenn die Komponente an einem Knochen gesichert ist, (a) eine Gelenkfläche bereitstellt oder (b) eine weitere Komponente aufnehmen kann, die eine Gelenkfläche bereitstellt.

4. Kit nach Anspruch 3, das eine zweite Komponente enthält, die eine Knochenanlagefläche mit einer Fläche, die eingerichtet ist, um an dem Knochen fest gesichert zu werden, und einen Gelenkabschnitt enthält, der eine Gelenkfläche zum Zusammenwirken mit der Gelenkfläche des Gelenkabschnitts, der von der Gelenkprothesenkomponente bereitgestellt wird, bereitstellen kann.

5. Kit nach Anspruch 1, wobei die Gelenkprothesenkomponente eine Hüftpfannenkomponente einer Hüftgelenkprothese ist.

## Revendications

1. Kit pour son utilisation dans la préparation d'un composant d'une prothèse articulaire pour son utilisation dans l'arthroplastie articulaire, le kit comprenant :
un composant (10) d'une prothèse articulaire ayant une partie de surface poreuse (18) formée d'un matériau métallique, la partie de surface poreuse ayant une surface poreuse de mise en prise avec l'os pour la fixation à un os pour former une articulation ;
un composé biologique (24) comprenant des cellules souches (26) adaptées pour être au moins partiellement dispersées dans la structure poreuse de la surface de mise en prise avec l'os de la partie de surface poreuse ;
un récipient (566) pour contenir un composé biologique ; et
une fixation (544) pour recevoir le composant, de sorte qu'il soit entouré par la fixation, la fixation comprenant une surface (562) qui se conforme étroitement à la surface de la partie poreuse du composant, la fixation ayant une entrée (570) et une sortie opposée (558) ; et
un dispositif (582) pour amener le composé biologique du récipient dans l'entrée de la fixation de sorte qu'il soit injecté dans la partie poreuse du composant et ainsi, que le composé biologique en excédent soit amené hors de la sortie de la fixation.

2. Kit selon la revendication 1, dans lequel les cellules souches comprennent au moins l'une des cellules parmi des cellules souches mésenchymateuses, des cellules progénitrices de tissu conjonctif de moelle osseuse et des cellules progénitrices de tissu conjonctif.

3. Kit selon la revendication 1, dans lequel le composant de prothèse articulaire a une partie d'articulation qui, lorsque le composant est fixé à un os, (a) constitue une surface d'articulation ou (b) peut recevoir un autre composant qui constitue une surface d'articulation.

4. Kit selon la revendication 3, qui comprend un deuxième composant comprenant une partie de mise en prise avec l'os ayant une surface adaptée pour être attachée fixement à l'os et une partie d'articulation qui peut constituer une surface d'articulation pour une coopération avec la surface d'articulation de la partie d'articulation constituée par ledit composant de prothèse articulaire.

5. Kit selon la revendication 1, dans lequel le composant de prothèse articulaire est un composant de cupule acétabulaire d'une prothèse d'articulation de hanche.
